# EUROPEAN PATENT SPECIFICATION

(11) **EP 2 246 052 B1**
(45) Date of publication and mention of the grant of the patent: **08.03.2017**
(21) Application number: 09706612.0
(22) Date of filing: 30.01.2009
(51) Int. Cl.: A61K 31/4164, A61K 9/20, A61K 9/00, A61P 13/10, A61K 9/50

(54) **ORALLY RAPIDLY DISINTEGRATING TABLET COMPRISING IMIDAFENACIN**
RASCH ORAL ZERFALLENDE TABLETTE UMFASSEND IMIDAFENACIN
COMPRIMÉ À ADMINISTRATION ORALE, À DÉSINTÉGRATION RAPIDE, COMPRENANT DE L'IMIDAFÉNACINE

(30) Priority: 31.01.2008 JP 2008020184
(43) Date of publication of application: 03.11.2010
(73) Proprietor: Kyorin Pharmaceutical Co., Ltd., Tokyo 101-8311 (JP)
(72) Inventor: ISHIZAKI, Toshihiro, Shimotsuga-gun Tochigi 329-0114 (JP); AOKI, Yoshinobu, Shimotsuga-gun Tochigi 329-0114 (JP)
(74) Representative: Vossius & Partner Patentanwälte Rechtsanwälte mbB
(86) International application number: PCT/JP2009/051652
(87) International publication number: WO 2009/096560

(56) References cited:
- EP-A1- 1 245 232
- EP-A1- 2 246 051
- WO-A1-00/47233
- WO-A1-01/34147
- WO-A1-2005/105045
- WO-A1-2006/080481
- JP-A- 2004 339 071

## Description

### Technical Field

The present invention relates to an orally rapidly disintegrating tablet comprising imidafenacin as active ingredient.

### Background Art

Imidafenacin is a compound having an antagonistic action in respect of muscarinic receptors M3 and M1 (see Patent Document 1 specified below), and it is provided as a medicament for treating overactive bladder (see Non-Patent Document 1 specified below). As pharmaceutical dosage form comprising imidafenacin as a main component, there have been known, for instance, an orally-administered solid pharmaceutical preparation and a transdermal therapeutic system, each of which contains imidafenacin (see Patent Documents 2 and specified below).

More specifically, Patent Document 2 discloses that the imidafenacin-containing pharmaceutical preparation is photosensitive and that the pharmaceutical preparation in the form of a tablet is correspondingly covered with a coating liquid which contains titanium oxide and iron sesquioxide to thus make the tablet photo-stable. However, Patent Document 2 does not disclose any tablet which is prepared by applying a coating agent onto an imidafenacin-containing granulated product prior to the compressing of the granulated product into tablet for the purpose of improving the photostability of the same.

In addition, Patent Document 3 relates to a pharmaceutical preparation of the transdermal therapeutic system type and therefore, the pharmaceutical preparation disclosed therein is different from the orally rapidly disintegrating tablet, in the dosage form. Incidentally, Patent Documents 4 and 5 disclose an orally administrable pharmaceutical preparation. However, the pharmaceutical preparations disclosed in Patent Documents 4 and 5 are those in the dosage form of the sustained release type. Accordingly a gel-forming substance, for instance, hydroxypropyl-methyl cellulose is used in the dosage form disclosed in Patent Document 4 in order to control the release rate of the drug. Moreover, the pharmaceutical preparation disclosed in Patent Document 5 is one in which the release of the drug is controlled through the use of a water-insoluble polymer or a higher alcohol.

On the other hand, there have intensively been conducted the development of pharmaceutical preparations while introducing improvements in manufacturing formulations into the development thereof, with the objective of improving the "Quality of Life (QOL)" of a patient. In this respect, most frequently or vigorously developed pharmaceutical preparations are orally rapidly disintegrating tablets, among others. The orally rapidly disintegrating tablet can instantaneously be disintegrated in the presence of even a small amount of saliva within the oral cavity and accordingly, the tablet can easily be administered to a patient and may be an optimum pharmaceutical preparation for the administration thereof to elderly peoples and children who cannot swallow the usual tablets with ease. In addition, the tablet of this type may have such a merit that it can be taken without the help of water and accordingly, there is not any limit in the place and/or time for the administration thereof.

However, it would be quite difficult to apply a coating, which comprises titanium oxide and iron sesquioxide, to the orally rapidly disintegrating tablet while ensuring the maintenance of the disintegration ability of the tablet and accordingly, there has not yet been proposed any report on the imidafenacin- containing orally rapidly disintegrating tablet which is photo-stable.

Non-Patent Document 1: Bioorg. Med. Chem., 1999, Vol. 7, pp. 1151-1161;
Patent Document 1: JP-A-7-215943;
Patent Document 2: WO 01/34147 A1 pamphlet;
Patent Document 3: WO 2006/082888 A1 pamphlet;
Patent Document 4: WO 2005/011682 A1 pamphlet;
Patent Document 5: WO 2006/0808481 A1 pamphlet.

### Disclosure of the Invention

### Problems That the Invention is to Solve

It is an object of the present invention to provide an imidafenacin-containing orally rapidly disintegrating tablet, which can be taken without any help of water and which is excellent in the photostability.

### Means for the Solution of the Problems

The inventors of this invention have conducted intensive studies to solve the foregoing problems, and have found that an intended orally rapidly disintegrating tablet, which is excellent in the photostability and which is also excellent in the disintegration property, can be obtained by the use of an imidafenacin-containing granulated product or imidafenacin particles coated with a specific coating agent and by the incorporation of a disintegrating agent into the outer layer of the tablet and have thus completed the present invention.

Accordingly, the present invention relates to an orally rapidly disintegrating tablet comprising a mixture of the following components: (1) an imidafenacin-containing granulated product or imidafenacin particles coated with a gastric juice-soluble polymer; and (2) a composition containing an excipient and a disintegrating agent, wherein said mixture is subjected to compression molding and wherein the gastric juice-soluble polymer is selected from methyl methacrylate-butyl methacrylate-dimethylamino-ethyl methacrylate copolymer or polyvinyl acetal diethylamino acetate.

This orally rapidly disintegrating tablet can be prepared by a method comprising the steps of covering an imidafenacin-containing granulated product or imidafenacin particles with povidone or a gastric juice-soluble polymer, blending the coated product with a composition containing an excipient and a disintegrating agent and then compression-molding the resulting mixture.

### Effects of the Invention

The present invention can thus provide an imidafenacin-containing orally rapidly disintegrating tablet which is excellent in the disintegration property within the oral cavity and in the photostability and the present invention thus permits the easy administration of a tablet to elderly peoples and children who cannot swallow the usual tablets with ease.

### Best Mode for Carrying Out the Invention

Now the present invention will be described below in more detail. In the specification of this patent application, the term "orally rapidly disintegrating tablet" means a solid pharmaceutical preparation for medical use, which can disintegrate, within the oral cavity and in the presence of saliva, within a term shorter than about 90 seconds, preferably shorter than about 60 seconds and further preferably shorter than 40 seconds, without any mastication. In addition, according to a preferred embodiment of the present invention, the orally rapidly disintegrating tablet does not show any sustained release property.

Imidafenacin used, as an active ingredient, in the orally rapidly disintegrating tablet of the present invention is 4-(2-methyl-1-imidazolyl)-2,2- diphenylbutylamide serving as a therapeutic agent for treating a patient suffering from urinary frequency and urinary incontinence, which has an anti-cholinergic effect selective for the bladder.

In the preparation of the orally rapidly disintegrating tablet of the present invention, an imidafenacin-containing granulated product or imidafenacin particles are coated with a coating agent.

The imidafenacin-containing granulated product can easily be prepared according to any granulation technique such as the dry granulation method, the agitation granulation method, the extrusion-granulation method, the fluidized bed-granulation method, the tumbling fluidized bed-granulation method, or the Spray drying granulation method. These granulation methods are well-known to one of ordinary skill in the art.

Applicable herein as such granulation techniques are preferably the fluidized bed-granulation method and the tumbling fluidized bed-granulation method. The preferred average particle size of the resulting granulated product ranges, for instance, from 0.1 to 350 *µ* m and more preferably 50 to 200 *µ* m, when it is determined according to the method as will be detailed later.

Additional components other than the medicinal component can further be incorporated into the granulated product or the powder so far as the intended effect of the present invention is not impaired. The excipient to be incorporated into the granulated product is preferably starch and more preferably partly pregelatinized starch, while taking into consideration the photostability of the resulting product.

In addition, the imidafenacin-containing granulated product may be dried after the granulation, from the viewpoint of the stability of the medicinal component used therein and easiness with which a tablet can be prepared. The applicable drying method herein is not restricted to any particular one so far as it can be used in the production of medicinal preparations.

The imidafenacin-containing granulated product and the imidafenacin particles are covered with a coating agent to make them photo-stable. The coating agent used in the present invention is selected from methyl methacrylate-butyl methacrylate-dimethylamino-ethyl methacrylate copolymer or polyvinyl acetal diethylamino acetate.

Povidone is a water-soluble polymer and it in general fulfills its function as a non-sustained release substance which is dissolved in the digestive tract existing between the throat and stomach, when the dosage form is orally disintegrated. Examples of such povidones include AIFUTACT K-30 (Dai-ichi Kogyo Seiyaku Co., Ltd.), Kollidon (BASF Japan Co., Ltd.), PLASDONE (ISP Japan Co., Ltd.), and POVIDONE (GOKYO Sangyo Co., Ltd.).

The gastric juice-soluble polymer is a polymeric compound having such properties that it is dissolved in the gastric juice and suitably used herein include, for instance, aminoalkyl methacrylate copolymer E [Trade Name: EUDRAGIT E100 (Rohm GmbH & Co. KG) and Trade Name: EUDRAGIT EPO (Rohm GmbH & Co. KG)], and polyvinyl acetal diethylamino acetate [Trade Name: AEA "SANKYO" (Sankyo LifeTech Co., Ltd.).

The content of the coating agent (selected from methyl methacrylate-butyl methacrylate-dimethylamino-ethyl methacrylate copolymer or polyvinyl acetal diethylamino acetate) present in the coated granulated product is not restricted to any specific one, but it preferably ranges, for instance, from about 0.001 to 10 parts by mass, more preferably about 0.01 to 1 part by mass and particularly preferably about 0.05 to 0.5 parts by mass per one part by mass of the uncoated granulated product.

In this respect, the method for applying the coating agent to the granulated product is not restricted to any specific one insofar as it can be used in the production of medicinal preparations.

In the orally rapidly disintegrating tablet according to the present invention, a composition containing an excipient and a disintegrating agent is used in combination with the coated imidafenacin-containing granulated product or the coated imidafenacin particles.

The excipient herein incorporated into the granulated product is not limited to any specific one, inasmuch as it can be used in the production of medicinal preparations and such an excipient can appropriately be used herein. Applicable herein as such excipients are, for instance, those disclosed in Dictionary of Drug Additives, edited by The Association of Drug Additives in Japan, published by YAKUJI-NIPPO Publishing Company (2007). Specific examples thereof suitably used herein include saccharides such as lactose and glucose, sugar alcohols such as D-sorbitol and mannitol, celluloses such as crystalline cellulose, and starches such as partially pre-gelatinized starch and corn starch. Preferably used herein as such excipients are sugar alcohols among others.

The disintegrating agent used in the present invention is not likewise limited to any specific one inasmuch as it can be used in the production of medicinal preparations and various kinds of disintegrating agents can be used herein. Suitably used herein as such disintegrating agents include, for instance, those disclosed in Dictionary of Drug Additives, edited by The Association of Drug Additives in Japan, published by YAKUJI-NIPPO Publishing Company (2007). Specific examples thereof include celluloses such as calcium carboxymethyl cellulose, hydroxypropyl cellulose having a low degree of substitution, croscarmellose sodium and methyl cellulose, crospovidone, with crospovidone being preferably used herein because of its immediate disintegration ability and the easiness of swallowing the resulting tablet (agreeableness to the palate). The amount of the disintegrating agent to be preferably incorporated into the granulated product falls within the range of from 1 to 10 % by mass and more preferably 2 to 6 % by mass on the basis of the mass of the tablet.

The orally rapidly disintegrating tablet according to the present invention may further comprise any additive usable in the production of medicinal preparations, if necessary. Specifically, such additives usable in the present invention include, for instance, those disclosed in Dictionary of Drug Additives, edited by The Association of Drug Additives in Japan, published by YAKUJI-NIPPO Publishing Company (2007) and specific examples thereof include lubricants such as stearic acid and metal salts thereof, as well as talc, light anhydrous silicic acid, hydrated silicon dioxide and sucrose esters of fatty acids; sweetening agents such as saccharides, sugar alcohols, aspartame, saccharin and salts thereof, glycyrrhizic acid and salts thereof, stevia and acesulfame potassium; corrigents (taste- and/or odor-improving agents) such as citric acid, sodium citrate, succinic acid, tartaric acid and fumaric acid; coloring agents such as iron sesquioxide, yellow iron sesquioxide, caramel, riboflavin and aluminum lakes; and perfumes such as menthol and orange oil extract.

In the preparation of the orally rapidly disintegrating tablet according to the present invention, the coated imidafenacin-containing granulated product or the imidafenacin particles are blended with a composition containing an excipient and a disintegrating agent. The amount of the composition containing an excipient and a disintegrating agent suitably used in the present invention ranges, for instance, from 1 to 50 parts by mass and preferably 3 to 25 parts by mass per one part by mass of the coated imidafenacin-containing granulated product or the imidafenacin particles.

The blending of the coated imidafenacin-containing granulated product or the imidafenacin particles with the composition containing an excipient and a disintegrating agent may be carried out using, for instance, an equipment such as a V-shaped blender, a diamond mixer and a drum mixer.

The mixture thus prepared is then subjected to compression molding to thus prepare the orally rapidly disintegrating tablet of the present invention.

The compression molding operation can suitably be carried out using the usual tableting machine such as a rotary tableting machine. In this connection, however, the shape of the orally rapidly disintegrating tablet of the present invention is not limited to any particular one so far as it never impairs the intended effects of the present invention. For instance, the foregoing imidafenacin-containing granulated product or the imidafenacin particles may be formed into even a specific shape such as an inside-bored shape, a polygonal shape or a concave shape. Moreover, the granulated product or the particles may likewise be formed into a flat-shaped tablet which is thin and has a large diameter in order to increase the contact area between the tongue and the tablet in the oral cavity to thus make the moisture within the oral cavity immediately penetrate into the interior of the tablet and to thereby improve the intraoral rapidly disintegration property of the tablet.

The pressure used in the compression molding operation falls within the range of, for instance, from 300 to 2,000 kg and preferably 600 to 1,000 kg and the compression molding can be implemented under the conditions of room temperature and 60% RH.

The present invention will now be described in more detail below with reference to the following Examples, Comparative Examples and Test Examples.

Below, the methods used for evaluating the imidafenacin-containing granulated product and the coated granules prepared from the same will be described:
[Determination of Particle Size Distribution]: The particle size was determined in a screening type particle size distribution-determining device (ATM; Sonic Shifter Co., Ltd.) while using sieves each having a mesh size of 75, 106, 150, 180, 212 or 355 *µ* m.

In addition, the average particle sizes (50% diameter) thereof were calculated on the basis of the results obtained in the determination of the particle size distribution.

The methods used for the various evaluation of the orally rapidly disintegrating tablet were as follows:
[Hardness Test]: The hardness of the tablet was determined using a tablet hardness meter (Okada Seiko Co., Ltd.). Each test was carried out using 5 tablets and each of the experimental results was the average of 5 measurements.
[Disintegration Test]: The disintegration time of the tablet was determined using a disintegration apparatus (TOYAMA Sangyo Co., Ltd.). Each test was carried out using 6 tablets and each of the experimental results was the average of 6 measurements. Water was used as a test liquid and the time required till each tablet was completely disintegrated and dissolved in water was determined.

Incidentally, the compounds represented by their trade names and used in the following Examples and Comparative Examples were as follows:
1. Trade Name: Starch 1500G (Colorcon Japan, LLC): Partially pre-gelatinized starch;
2. Trade Names: CEOLUS PH-301 and PH-102 (ASAHIKASEI Chemicals Co., Ltd.): Crystalline celluloses;
3. Trade Name: Kollidon 90F (BASF Japan Ltd.): Povidone;
4. Trade Name: EUDRAGIT EPO (Rohm GmbH & Co. KG): Methyl methacrylate-butyl methacrylate-dimethylamino-ethyl methacrylate copolymer or aminoalkyl methacrylate copolymer E;
5. Trade Name: HPC-SSL (Nippon Soda Co., Ltd.): Hydroxypropyl cellulose;
6. Trade Name: Neusilin US2 (Fuji Chemical Industry Co., Ltd.): magnesium metasilicate aluminate;
7. Trade Name: magnesium stearate (derived from vegetable) (Taihei Chemical Industry Co., Ltd.);
8. Trade Name: PEARLITOL (ROQUETTE JAPAN Co., Ltd.): D-Mannitol;
9. Trade Name: Kollidon CL-F (BASF Japan Ltd.): Crospovidone;
10. Trade Name: POLYPLASDONE XL-10 (ISP Company): Crospovidone;
11. Trade Name: CARPREX #67 (DSL Japan Co., Ltd.): Hydrated silicon dioxide;
12. Trade Name: ETHOCEL 7 Premium (Nissin Chemical Industry Co., Ltd.): Ethyl cellulose;
13. Trade Name: TC-5RW (Shin-Etsu Chemical Co., Ltd.): Hydroxy-propylmethyl cellulose.
14. Trade Name: AEA (Sankyo LifeTech Co., Ltd.): Polyvinyl acetal diethylamino acetate;
15. Trade Name: Kollidon 25 (BASF Japan Ltd.): Povidone;

### Example 1

There were dissolved 4.0 g of imidafenacin and 2 g of Kollidon 90F BASF Japan Ltd.) in a mixed liquid comprising 118.2 g of purified water and 275.8 g of ethanol. Then, there was charged 394 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 15 g/min, air pressure used for spray: 0.1 MPa, air supply temperature: 70°C) to thus give an imidafenacin-containing granulated product.

Separately, there were dissolved 60 g of EUDRAGIT EPO (Rohm GmbH & Co. KG) as a gastric juice-soluble polymer and 30 g of magnesium stearate (derived from vegetable) (Taihei Chemical Industry Co., Ltd.) in a mixed liquid comprising 273 g of purified water and 637 g of ethanol.

Then, there was charged 300 g of the imidafenacin-containing granulated product prepared above into a tumbling fluidized bed granulator (NQ-160, DALTON Co., Ltd.) and the solution thus prepared was then applied to the granulated product according to the top-spray technique (amount of liquid to be sprayed: 20 g/min, air pressure for spray: 0.15 MPa, air supply temperature: 80"C) to thus give coated granules.

Furthermore, there were blended 5.2 g of the resulting coated granules, 63.76 g of PEARLITOL (ROQUETTE JAPAN Co., Ltd.), 2.16 g of Kollidon CL-F (BASF Japan Ltd.) and 0.16 g of CARPREX #67 (DSL Japan Co., Ltd.), together, followed by the addition, to the resulting blend, of 0.72 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a single tableting machine under a compressing pressure of 670 kg. The resulting tablets were inspected for the hardness and as a result, it was found to be 5.1 kg (n=5).

### Example 2

There were dissolved 25.0 g of imidafenacin and 12.5 g of Kollidon 90F (BASF Japan Ltd.) in a mixed liquid comprising 738.75 g of purified water and 1723.75 g of ethanol. Then, there was charged 4962.5 g of Starch 1500G (Colorcon Japan, LLC.) into a fluidized bed granulator (FL-5 Freund Industry Co., Ltd.) and the foregoing solution was then applied to (coated) the starch according to the top-spray technique (amount of liquid to be sprayed: 100 g/min, air pressure used for spray: 0.3 MPa, air supply temperature: 70°C) to thus give an imidafenacin-containing granulated product (average particle size: 128 *µ* m). Separately, there were dissolved 750 g of EUDRAGIT EPO (Rohm GmbH & Co. KG) and 375 g of magnesium stearate (derived from vegetable) (Taihei Chemical Industry Co., Ltd.) in a mixed liquid comprising 3412.5 g of purified water and 7962.5 g of ethanol. Then, there was charged 3750 g of the imidafenacin-containing granulated product prepared above into a fluidized bed granulator (FL-5, Freund Industry Co., Ltd.) and the solution thus prepared was then applied to the granulated product according to the top-spray technique (amount of liquid to be sprayed: 100 g/min, air pressure for spray: 0.3 MPa, air supply temperature: 80°C) to thus give coated granules (average particle size: 117 *µ* m).

Furthermore, there were blended 2600 g of the resulting coated granules, 14640 g of PEARLITOL (ROQUETTE JAPAN Co., Ltd.), 540 g of Kollidon CL-F (BASF Japan Ltd.) and 40 g of CARPREX #67 (DSL Japan Co., Ltd.), together, followed by the addition, to the resulting blend, of 180 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 9 kN. The resulting tablets were inspected for the hardness and the disintegration time and as a result, they were found to be 5.6 kg (n=5) and 12 seconds (n=6), respectively.

### Example 3

There were dissolved 20.0 g of imidafenacin and 10.0 g of Kollidon 90F (BASF Japan Ltd.) in a mixed liquid comprising 591 g of purified water and 1379 g of ethanol. Then, there was charged 4970 g of Starch 1500G (Colorcon Japan, LLC.) into a fluidized bed granulator (FL-5 Freund Industry Co., Ltd.) and the foregoing solution was then applied to (coated) the starch according to the top-spray technique (amount of liquid to be sprayed: 100 g/min, air pressure used for spray: 0.3 MPa, air supply temperature: 70°C) to thus give an imidafenacin-containing granulated product (average particle size: 124 *µ* m). Separately, there were dissolved 750 g of EUDRAGIT EPO (Rohm GmbH & Co. KG) and 375 g of magnesium stearate (derived from vegetable) (Taihei Chemical Industry Co., Ltd.) in a mixed liquid comprising 3412.5 g of purified water and 7962.5 g of ethanol. Then, there was charged 3750 g of the imidafenacin-containing granulated product prepared above into a fluidized bed granulator (FL-5, Freund Industry Co., Ltd.) and the solution thus prepared was then applied to the granulated product according to the top-spray technique (amount of liquid to be sprayed: 100 g/min, air pressure for spray: 0.3 MPa, air supply temperature: 80°C) to thus give coated granules (average particle size: 120 *µ* m).

Furthermore, there were blended 3250 g of the resulting coated granules, 13990 g of PEARLITOL (ROQUETTE JAPAN Co., Ltd.), 540 g of Kollidon CL-F (BASF Japan Ltd.) and 40 g of CARPREX #67 (DSL Japan Co., Ltd.), together, followed by the addition, to the resulting blend, of 180 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 9 kN. The resulting tablets were inspected for the hardness and the disintegration time and as a result, they were found to be 4.9 kg (n=5) and 10 seconds (n=6), respectively.

### Example 4

There were dissolved 16.7 g of imidafenacin and 8.3 g of Kollidon 90F (BASF Japan Ltd.) in a mixed liquid comprising 438.5 g of purified water and 1151.5 g of ethanol. Then, there was charged 4975 g of Starch 1500G (Colorcon Japan, LLC.) into a fluidized bed granulator (FL-5 Freund Industry Co., Ltd.) and the foregoing solution was then applied to (coated) the starch according to the top-spray technique (amount of liquid to be sprayed: 100 g/min, air pressure used for spray: 0.3 MPa, air supply temperature: 70°C) to thus give an imidafenacin-containing granulated product (average particle size: 119 *µ* m). Separately, there were dissolved 750 g of EUDRAGIT EPO (Rohm GmbH & Co. KG) and 375 g of magnesium stearate (derived from vegetable) (Taihei Chemical Industry Co., Ltd.) in a mixed liquid comprising 3412.5 g of purified water and 7962.5 g of ethanol. Then, there was charged 3750 g of the imidafenacin-containing granulated product prepared above into a fluidized bed granulator (FL-5, Freund Industry Co., Ltd.) and the solution thus prepared was then applied to the granulated product according to the top-spray technique (amount of liquid to be sprayed: 100 g/min, air pressure for spray: 0.3 MPa, air supply temperature: 80°C) to thus give coated granules (average particle size: 115 *µ* m).

Furthermore, there were blended 3900 g of the resulting coated granules, 13340 g of PEARLITOL COQUETTE JAPAN Co., Ltd.), 540 g of Kollidon CL-F (BASF Japan Ltd.) and 40 g of CARPREX #67 (DSL Japan Co., Ltd.), together, followed by the addition, to the resulting blend, of 180 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 9 kN. The resulting tablets were inspected for the hardness and the disintegration time and as a result, they were found to be 5.2 kg (n=5) and 16 seconds (n=6), respectively.

### Example 5

There were dissolved 25.0 g of imidafenacin and 12.5 g of Kollidon 90F (BASF Japan Ltd.) in a mixed liquid comprising 738.75 g of purified water and 1723.75 g of ethanol. Then, there was charged 2500 g of Starch 1500G (Colorcon Japan, LLC.) and 2462.5 g of CEOLUS PH-301 (ASAHIKASEI Chemicals Co., Ltd.) into a fluidized bed granulator (FL-5 Freund Industry Co., Ltd.) and the foregoing solution was then applied to (coated) the starch-cellulose mixture according to the top-spray technique (amount of liquid to be sprayed: 100 g/min, air pressure used for spray: 0.3 MPa, air supply temperature: 70°C) to thus give an imidafenacin-containing granulated product (average particle size: 85 *µ* m). Separately, there were dissolved 750 g of EUDRAGIT EPO (Rohm GmbH & Co. KG) and 375 g of magnesium stearate (derived from vegetable) (Taihei Chemical Industry Co., Ltd.) in a mixed liquid comprising 3412.5 g of purified water and 7962.5 g of ethanol. Then, there was charged 3750 g of the imidafenacin-containing granulated product prepared above into a fluidized bed granulator (FL-5, Freund Industry Co., Ltd.) and the solution thus prepared was then applied to the granulated product according to the top-spray technique (amount of liquid to be sprayed: 100 g/min, air pressure for spray: 0.3 MPa, air supply temperature: 80°C) to thus give coated granules (average particle size: 118 *µ* m).

Furthermore, there were blended 2600 g of the resulting coated granules, 14640 g of PEARLITOL (ROQUETTE JAPAN Co., Ltd.), 540 g of Kollidon CL-F (BASF Japan Ltd.) and 40 g of CARPREX #67 (DSL Japan Co., Ltd.), together, followed by the addition, to the resulting blend, of 180 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 7.7 kN. The resulting tablets were inspected for the hardness and the disintegration time and as a result, they were found to be 5.2 kg (n=5) and 12 seconds (n=6), respectively.

### Example 6

There were dissolved 25.0 g of imidafenacin, 125 g of EUDRAGIT EPO (Rohm GmbH & Co. KG) and 62.5 g of magnesium stearate (derived from vegetable) (Taihei Chemical Industry Co., Ltd.) in a mixed liquid comprising 915 g of purified water and 1372.5 g of ethanol. Then, there was charged 4787.5 g of Starch 1500G (Colorcon Japan, LLC.) into a fluidized bed granulator (FL-5 Freund Industry Co., Ltd.) and the foregoing solution was then applied to (coated) the starch according to the top-spray technique (amount of liquid to be sprayed: 1.00 g/min, air pressure used for spray: 0.3 MPa, air supply temperature: 70 °C) to thus give an imidafenacin-containing granulated product (average particle size: 115 *µ* m). Separately, there were dissolved 940 g of EUDRAGIT EPO (Rohm GmbH & Co. KG) and 470 g of magnesium stearate (derived from vegetable) (Taihei Chemical Industry Co., Ltd.) in a mixed liquid comprising 5702.8 g of purified water and 8554.2 g of ethanol. Then, there was charged 4700 g of the imidafenacin- containing granulated product prepared above into a fluidized bed granulator (FL-5, Freund Industry Co., Ltd.) and the solution thus prepared was then applied to the granulated product according to the top-spray technique (amount of liquid to be sprayed: 100 g/min, air pressure for spray: 0.3 MPa, air supply temperature: 80°C) to thus give coated granules (average particle size: 123 *µ* m).

Furthermore, there were blended 2600 g of the resulting coated granules, 14640 g of PEARLITOL (ROQUETTE JAPAN Co., Ltd.), 540 g of Kollidon CL-F (BASF Japan Ltd.) and 40 g of CARPREX #67 (DSL Japan Co., Ltd.), together, followed by the addition, to the resulting blend, of 180 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 8.6 kN. The resulting tablets were inspected for the hardness and the disintegration time and as a result, they were found to be 5.6 kg (n=5) and 12 seconds (n=6), respectively.

### Comparative Example 1

There were dissolved 4.0 g of imidafenacin and 2 g of Kollidon 90F (BASF Japan Ltd.) in a mixed liquid comprising 197 g of purified water and 197 g of ethanol. Then, there was charged 394 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 15 g/min, air pressure used for spray: 0.1 MPa, air supply temperature: 50°C) to thus give an imidafenacin-containing granulated product (average particle size: 134 *µ* m).

Furthermore, there were blended 25 g of the resulting imidafenacin-containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 800 kg. The resulting tablets were inspected for the hardness and as a result, it was found to be 4.7 kg (n=5).

### Comparative Example 2

There were dissolved 4.0 g of imidafenacin and 4 g of Kollidon 90F (BASF Japan Ltd.) in a mixed liquid comprising 196 g of purified water and 196 g of ethanol. Then, there was charged 392 g of PEARLITOL (ROQUETTE JAPAN Co., Ltd.) into a tumbling fluidized bed granulator (NQ-160, DALTON Co., Ltd.) and the foregoing solution was then applied to (coated) the sugar alcohol according to the top-spray technique (amount of liquid to be sprayed: 15 g/min, air pressure used for spray: 0.1 MPa, air supply temperature: 50 °C) to thus give an imidafenacin-containing granulated product.

Furthermore, there were blended 25 g of the resulting imidafenacin- containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 1,000 kg. The resulting tablets were inspected for the hardness and as a result, it was found to be 7.2 kg (n=5).

### Comparative Example 3

There was dissolved 2.0 g of imidafenacin in a mixed liquid comprising 100 g of purified water and 100 g of ethanol. Then, there was charged 200 g of Neucillin US2 (Fuji Chemical Industry Co., Ltd.) into a tumbling fluidized bed granulator (NQ-160, DALTON Co., Ltd.) and the foregoing solution was then applied to (coated) the magnesium salt according to the top-spray technique (amount of liquid to be sprayed: 15 g/min, air pressure used for spray: 0.1 MPa, air supply temperature: 50°C) to thus give an imidafenacin-containing granulated product.

Furthermore, there were blended 25 g of the resulting imidafenacin- containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 700 kg. The resulting tablets were inspected for the hardness and as a result, it was found to be 7.2 kg (n=5).

### Comparative Example 4

There was dissolved 4.0 g of imidafenacin and 40 g of Polyplasdone XL-10 (ISP Company) in a mixed liquid comprising 178 g of purified water and 178 g of ethanol. Then, there was charged 356 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 15 g/min, air pressure used for spray: 0.1 MPa, air supply temperature: 50°C) to thus give an imidafenacin-containing granulated product.

Furthermore, there were blended 25 g of the resulting imidafenacin- containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 850 kg. The resulting tablets were inspected for the hardness and as a result, it was found to be 4.8 kg (n=5).

### Comparative Example 5

There was dissolved 4.0 g of imidafenacin and 40 g of HPC-SSL (Nippon Soda Co., Ltd.) in a mixed liquid comprising 178 g of purified water and 178 g of ethanol. Then, there was charged 356 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 15 g/min, air pressure used for spray: 0.1 MPa, air supply temperature: 50°C) to thus give an imidafenacin-containing granulated product.

Furthermore, there were blended 25 g of the resulting imidafenacin- containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 1,000 kg. The resulting tablets were inspected for the hardness and as a result, it was found to be 6.7 kg (n=5).

### Comparative Example 6

There was dissolved 3.0 g of imidafenacin and 9 g of Kollidon 90F (BASF Japan Ltd.) in a mixed liquid comprising 144 g of purified water and 144 g of ethanol. Then, there was charged 288 g of Polyplasdone XL-10 (ISP Company) into a tumbling fluidized bed granulator (NQ-160, DALTON Co., Ltd.) and the foregoing solution was then applied to (coated) Polyplasdone XL-10 according to the top-spray technique (amount of liquid to be sprayed: 15 g/min, air pressure used for spray: 0.1 MPa, air supply temperature: 50°C) to thus give an imidafenacin-containing granulated product.

Furthermore, there were blended 25 g of the resulting imidafenacin- containing granulated product, 374.5 g of PEARLITOL (ROQUETTE JAPAN Co., Ltd.), 45 g of Polyplasdone XL-10 (ISP Company) and 1 g of CARPREX #67 (DSL Japan Co., Ltd.), together, followed by the addition, to the resulting blend, of 4.5 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 1,000 kg. The resulting tablets were inspected for the hardness and as a result, it was found to be 6.7 kg (n=5).

### Test Example 1

The pharmaceutical preparations prepared in the foregoing Examples 1 to 6 and Comparative Examples 1 to 6 were subjected to a test for evaluating the photostability thereof. Each sample was irradiated with D65 lamp at an illuminance of 4500 Lx over about 12 days. The results of the amount of degradation products formed during the term are summarized in the following Table 1. Incidentally, the quantitative analysis of the degradation products was carried out according to the high performance liquid chromatography technique (HPLC technique).

### HPLC Technique:

Column Used: Octadecylsilanized silica gel (average particle size: 5 *µ* m; 4.6 mm (inner diameter) × 250 mm (length)) (GL Science Co., Ltd. under the trade name of Inertsil ODS-3,);
   Liquid A: Diethylamine was added to a diluted phosphoric acid (1→200) and the pH value thereof was controlled to 6.0;
   Liquid B: Acetonitrile of liquid chromatography grade;
   Liquid C: Methanol of liquid chromatography grade;
Carrier Liquid: The concentration gradient was controlled by changing the mixing ratio of the liquid A, liquid B and liquid C;
Detector: UV;
Wavelength Used for Measurement: 220 nm.

**[Table 1]**

| Sample | Before Irradiation with Light | | After Irradiation with Light | |
|---|---|---|---|---|
| | Indiv. (max.), % | Total, % | Indiv. (max.), % | Total, % |
| Ex. 1 | 0.07 | 0.1 | 0.10 | 0.4 |
| Ex. 2 | 0.15 | 0.3 | 0.15 | 0.5 |
| Ex. 3 | 0.09 | 0.2 | 0.23 | 0.7 |
| Ex. 4 | 0.12 | 0.2 | 0.26 | 0.9 |
| Ex. 5 | 0.12 | 0.3 | 0.27 | 1.2 |
| Ex. 6 | N.D. | N.D. | 0.18 | 0.6 |
| Comp. Ex. 1 | 0.04 | 0.0 | 1.67 | 3.1 |
| Comp. Ex. 2 | N.D. | N.D. | 3.19 | 6.0 |
| Comp. Ex. 3 | 0.05 | 0.1 | 18.76 | 36.5 |
| Comp. Ex. 4 | 0.12 | 0.2 | 2.97 | 6.0 |
| Comp. Ex. 5 | N.D. | N.D. | 3.69 | 7.7 |
| Comp. Ex. 6 | 0.06 | 0.1 | 3.11 | 5.9 |

As will be seen from the data listed in the foregoing Table 1, the pharmaceutical preparations prepared in Comparative Examples 1 to 6 in which the imidafenacin-containing granulated products are free of any coating layer are accompanied by the formation of large amounts of degradation products and the maximum amount of the individual degradation product exceeds 1%. Contrary to this, the maximum amount of the individual degradation product does not exceed 1%, in case of the pharmaceutical preparations prepared in Examples 1 to 6 which comprise the granulated products each covered with EUDRAGIT EPO.

### Example 7

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Separately, there were dissolved 60 g of EUDRAGIT EPO (Rohm GmbH & Co. KG) and 30 g of magnesium stearate (derived from vegetable) (Taihei Chemical Industry Co., Ltd.) in a mixed liquid comprising 364 g of purified water and 546 g of ethanol. Then, there was charged 300 g of the imidafenacin-containing granulated product prepared above into a tumbling fluidized bed granulator (NQ-160, DALTON Co., Ltd.) and the solution thus prepared was then applied to the granulated product according to the side-spray technique (amount of liquid to be sprayed: 20 g/min, air pressure for spray: 0.15 MPa, air supply temperature: 70°C) to thus give coated granules.

Furthermore, there were blended 78.0 g of the resulting coated granules, 439.2 g of PEARLITOL (ROQUETTE JAPAN Co., Ltd.), 16.2 g of Kollidon CL-F (BASF Japan Ltd.) and 1.2 g of CARPREX #67 (DSL Japan Co., Ltd.), together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 800 kg. The resulting tablets were inspected for the hardness and as a result, it was found to be 5.2 kg (n=5).

### Example 8

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Separately, there were dissolved 60 g of AEA (Sankyo LifeTech Co., Ltd.), as a gastric juice-soluble polymer, and 30 g of magnesium stearate (derived from vegetable) (Taihei Chemical Industry Co., Ltd.) in a mixed liquid comprising 91 g of purified water and 819 g of ethanol. Then, there was charged 300 g of the imidafenacin-containing granulated product prepared above into a tumbling fluidized bed granulator (NQ-160, DALTON Co., Ltd.) and the solution thus prepared was then applied to the granulated product according to the side-spray technique (amount of liquid to be sprayed: 20 g/min, air pressure for spray: 0.2 MPa, air supply temperature: 60°C) to thus give coated granules.

Furthermore, there were blended 78.0 g of the resulting coated granules, 439.2 g of PEARLITOL (ROQUETTE JAPAN Co., Ltd.), 16.2 g of KOLLIDON CL-F (BASF Japan Ltd.) and 1.2 g of CARPREX #67 (DSL Japan Co., Ltd.), together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 800 kg. The resulting tablets were inspected for the hardness and as a result, it was found to be 4.8 kg (n=5).

### Example 9

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Separately, there were dissolved 60 g of Kollidon 25 (BASF Japan Ltd.) and 30 g of magnesium stearate (derived from vegetable) (Taihei Chemical Industry Co., Ltd.) in a mixed liquid comprising 364 g of purified water and 546 g of ethanol. Then, there was charged 300 g of the imidafenacin-containing granulated product prepared above into a tumbling fluidized bed granulator (NQ-160, DALTON Co., Ltd.) and the solution thus prepared was then applied to the granulated product according to the side-spray technique (amount of liquid to be sprayed: 15 g/min, air pressure for spray: 0.15 MPa, air supply temperature: 70°C) to thus give coated granules.

Furthermore, there were blended 78.0 g of the resulting coated granules, 439.2 g of PEARLITOL (ROQUETTE JAPAN Co., Ltd.), 16.2 g of Kollidon CL-F (BASF Japan Ltd.) and 1.2 g of CARPREX #67 (DSL Japan Co., Ltd., together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 820 kg. The resulting tablets were inspected for the hardness and as a result, it was found to be 4.9 kg (n=5).

### Comparative Example 7

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Separately, there were dissolved 60 g of ETHOCEL 7 Premium (Nissin Chemical Industry Co., Ltd.) and 30 g of magnesium stearate (derived from vegetable) (Taihei Chemical Industry Co., Ltd.) in a mixed liquid comprising 91 g of purified water and 819 g of ethanol. Then, there was charged 300 g of the imidafenacin-containing granulated product prepared above into a tumbling fluidized bed granulator (NQ-160, DALTON Co., Ltd.) and the solution thus prepared was then applied to the granulated product according to the side-spray technique (amount of liquid to be sprayed: 20 g/min, air pressure for spray: 0.15 MPa, air supply temperature: 60°C) to thus give coated granules.

Furthermore, there were blended 78.0 g of the resulting coated granules, 439.2 g of PEARLITOL (ROQUETTE JAPAN Co., Ltd.), 16.2 g of Kollidon CL-F (BASF Japan Ltd.) and 1.2 g of CARPREX #67 (DSL Japan Co., Ltd.), together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 830 kg. The resulting tablets were inspected for the hardness and as a result, it was found to be 5.5 kg (n=5).

### Comparative Example 8

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Separately, there were dissolved 60 g of TC-5RW (Shin-Etsu Chemical Co., Ltd.) and 30 g of magnesium stearate (derived from vegetable) (Taihei Chemical Industry Co., Ltd.) in a mixed liquid comprising 364 g of purified water and 546 g of ethanol. Then, there was charged 300 g of the imidafenacin-containing granulated product prepared above into a tumbling fluidized bed granulator (NQ-160, DALTON Co., Ltd.) and the solution thus prepared was then applied to the granulated product according to the side-spray technique (amount of liquid to be sprayed: 15 g/min, air pressure for spray: 0.15 MPa, air supply temperature: 70°C) to thus give coated granules.

Furthermore, there were blended 78.0 g of the resulting coated granules, 439.2 g of PEARLITOL (ROQUETTE JAPAN Co., Ltd.), 16.2 g of Kollidon CL-F (BASF Japan Ltd.) and 1.2 g of CARPREX #67 (DSL Japan Co., Ltd.), together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 800 kg. The resulting tablets were inspected for the hardness and as a result, it was found to be 4.4 kg (n=5).

### Comparative Example 9

There were dissolved 4.0 g of imidafenacin and 2 g of Kollidon 90F (BASF Japan Ltd.) in a mixed liquid comprising 157.6 g of purified water and 236.4 g of ethanol. Then, there was charged 794 g of Starch 1500G (Colorcon Japan, LLC.) into a tumbling fluidized bed granulator (NQ-160, DALTON Co., Ltd.) and the foregoing solution was then applied to (coated) the starch according to the top-spray technique (amount of liquid to be sprayed: 18 g/min, air pressure used for spray: 0.1 MPa, air supply temperature: 70°C) to thus give an imidafenacin-containing granulated product. Furthermore, there were blended 60.0 g of the resulting imidafenacin-containing granulated product, 457.2 g of PEARLITOL (ROQUETTE JAPAN Co., Ltd.), 16.2 g of KOLLIDON CL-F (BASF Japan Ltd.) and 1.2 g of CARPREX #67 (DSL Japan Co., Ltd.), together, followed by the addition, to the resulting blend, of 5.4 g of magnesium stearate (derived from vegetable) (Taihei Chemical Industry Co., Ltd.), the blending of these components and the subsequent forming of the resulting blend into tablets each having a weight of 180 mg and an imidafenacin content of 0.1 mg using a rotary tableting machine under a compressing pressure of 790 kg. The resulting tablets were inspected for the hardness and as a result, it was found to be 4.9 kg (n=5).

### Test Example 2

The pharmaceutical preparations prepared in the foregoing Examples 7 to 11 and Comparative Examples 7 to 9 were subjected to a test for evaluating the photostability thereof. Each sample was irradiated with D65 lamp at an illuminance of 2000 to 4500 Lx over about 18 days. The results of the amount of degradation products formed during the term are summarized in the following Table 2. Incidentally, the quantitative analysis of the degradation products was carried out according to the high performance liquid chromatography (HPLC technique).

### HPLC Technique:

Column Used: Octadecylsilanized silica gel (average particle size: 5 *µ* m; 4.6 mm (inner diameter) × 150 mm (length)) (GL Science Co., Ltd. under the trade Name of Inertsil ODS-3);
   Liquid A: Sodium octane sulfonate (1.08 g) was dissolved in a diluted phosphoric acid (1→1000), followed by the adjustment of the final volume to 1000 mL;
   Liquid B: Acetonitrile of liquid chromatography grade;
Carrier Liquid: The concentration gradient was controlled by changing the mixing ratio of the liquid A to the liquid B;
Detector: UV;
Wavelength Used for Measurement: 220 nm.

**[Table 2]**

| Sample | Before Irradiation with Light | | After Irradiation with Light | |
|---|---|---|---|---|
| | Indiv. (max.), % | Total, % | Indiv. (max.), % | Total, % |
| Ex. 7 | 0.08 | 0.1 | 0.19 | 0.5 |
| Ex. 8 | N.D. | -- | 0.3 | 0.7 |
| Ex. 9 | 0.05 | 0.1 | 1.49 | 3 |
| Comp. Ex. 7 | 0.35 | 0.5 | 8.69 | 21.1 |
| Comp. Ex. 8 | 0.04 | 0.1 | 3.24 | 7.1 |
| Comp. Ex. 9 | 0.03 | 0.1 | 7.65 | 13.7 |

As will be seen from the data listed in the foregoing Table 2, the pharmaceutical preparations prepared in Comparative Examples 7 to 9 each are accompanied by the generation of a large amount of degradation products and the maximum amount of the individual degradation product exceeds 3%. Contrary to this, the maximum amount of the individual degradation product does not exceed 2%, in case of the pharmaceutical preparations prepared in Examples 7 to 9 in which the granulated products each are covered with a coating agent.

### Industrial Applicability

The present invention permits the preparation of an imidafenacin-containing orally rapidly disintegrating tablet which is excellent in the photostability and the physical burden of a patient upon the oral administration of the tablet can considerably be lightened or relieved and the QOL of the patient can thus be improved.

## Claims

1. An orally rapidly disintegrating tablet comprising a mixture of the following components:
(1) an imidafenacin-containing granulated product or imidafenacin particles, coated with a gastric juice-soluble polymer; and
(2) a composition containing an excipient and a disintegrating agent,
wherein said mixture is subjected to compression molding and wherein the gastric juice-soluble polymer is selected from methyl methacrylate-butyl methacrylate-dimethylamino-ethyl methacrylate copolymer or polyvinyl acetal diethylamino acetate.

2. The orally rapidly disintegrating tablet according to claim 1, wherein the gastric juice-soluble polymer is methyl methacrylate-butyl methacrylate-dimethylamino-ethyl methacrylate copolymer.

3. The orally rapidly disintegrating tablet according to claim 1 or 2, wherein the granulated product comprises an excipient and a binder.

## Patentansprüche

1. Eine oral rasch zerfallende Tablette umfassend ein Gemisch folgender Komponenten:
(1) ein Imidafenacin enthaltendes, granuliertes Produkt oder Imidafenancin Teilchen, beschichtet mit einem magensaftlöslichem Polymer; und
(2) eine Zusammensetzung enthaltend einen Exzipienten und ein Sprengmittel, wobei das Gemisch Formpressen ausgesetzt wird und wobei das magensaftlösliche Polymer aus Methylmethacrylat-Butylmethacrylat-Dimethylaminoethylmethacrylat-Copolymer oder Polyvinylacetaldiethylaminoacetat ausgewählt ist.

2. Die oral rasch zerfallende Tablette gemäß Anspruch 1, worin das magensaftlösliche Polymer aus Methylmethacrylat-Butylmethacrylat-Dimethylaminoethylmethacrylat-Copolymer ist.

3. Die oral rasch zerfallende Tablette gemäß Anspruch 1 oder 2, wobei das granulierte Produkt einen Exzipienten und ein Bindemittel umfasst.

## Revendications

1. Comprimé se désintégrant rapidement par voie orale comprenant un mélange des composants suivants :
(1) un produit granulé contenant de l'imidafénacine ou des particules d'imidafénacine enrobées d'un polymère soluble dans les sucs gastriques ; et
(2) une composition contenant un excipient et un agent délitant,
dans lequel ledit mélange est soumis à un moulage par compression et dans lequel le polymère soluble dans les sucs gastriques est choisi parmi un copolymère de méthacrylate de méthyle/méthacrylate de butyle/ méthacrylate de diméthylaminoéthyle et le poly(diéthylaminoacétate de vinylacétal).

2. Comprimé se désintégrant rapidement par voie orale selon la revendication 1, dans lequel le polymère soluble dans les sucs gastriques est un copolymère de méthacrylate de méthyle/méthacrylate de butyle/ méthacrylate de diméthylaminoéthyle.

3. Comprimé se désintégrant rapidement par voie orale selon la revendication 1 ou 2, dans lequel le produit granulé comprend un excipient et un liant.
